# EUROPEAN PATENT APPLICATION

(11) **EP 3 654 347 A1**
(43) Date of publication of application: **20.05.2020**
(21) Application number: 19195359.5
(22) Date of filing: 04.09.2019
(51) Int. Cl.: G16H 40/63, G16H 40/67, G16H 50/20, G16H 80/00, A61B 5/00

(54) **A SYSTEM AND METHOD FOR PERSONALIZED MONITORING OF LIFE-THREATENING HEALTH CONDITIONS IN PATIENTS WITH CHRONIC KIDNEY DISEASE**

(30) Priority: 16.11.2018 LT 2018549
(71) Applicant: Kaunas University of Technology, 44249 Kaunas (LT)
(72) Inventor: Marozas, Vaidotas, Kaunas (LT); Petrenas, Andrius, Kaunas (LT); Paliakaite, Birute, Kaunas (LT); Lukosevicius, Arunas, Kaunas (LT); Jegelevicius, Darius, Kaunas (LT); Solosenko, Andrius, Kaunas (LT); Daukantas, Saulius, Kaunas (LT); Rapalis, Andrius, Kaunas (LT); Kusleikaite-Pere, Neda, Kaunas (LT); Bumblyte, Inga Arune, Kaunas (LT)
(74) Representative: Klimaitiene, Otilija

(57) **Abstract**

This description provides a system and a method for recognizing and monitoring a patient's life-threatening conditions due to pathological electrolyte fluctuations in blood serum and episodes of arrhythmias. The system comprises a patient-worn device with integrated biosignal sensors; real-time signal processing modules integrated in the worn device; and a sudden-death-risk-assessment module that is used in the server, personal computer, or smart device. Life-threatening arrhythmias are recognized by real-time analysis of the continuously recorded photoplethysmogram signal, and electrolyte fluctuations are monitored by analysis of a short electrocardiogram signal recorded by integrated biopotential sensors. The system user shall have the possibility to enter, by means of a smart device, other information related to his or her medical condition that is visible on the physician's smart device. In his smart device, the doctor sees detailed information about the patient's state of health and the risk of life-threatening conditions.

The system is designed to monitor the health of patients with chronic kidney disease, who are subject to hemodialysis, but it can also be used to home-monitor other patients after serious illness. The test means are non-invasive, reusable on a continuous basis, do not restrict patient movements, and wireless; test results and feedback are transmitted over the internet.

## Description

### TECHNICAL FIELD

The invention relates to the field of medical equipment and particularly it is a system and method for monitoring life-threatening conditions in patients with chronic kidney disease.

### BACKGROUND ART

Described are the system and the method for the patient-friendly recognition and monitoring of life-threatening medical conditions in patients with chronic kidney disease, who are subject to hemodialysis. In the final phase of chronic kidney disease, one third of deaths have been sudden deaths due to life-threatening cardiac arrhythmias, a large proportion of which occur on the last day of the long interval between hemodialysis procedures; this is associated with electrolyte imbalance in the blood. Electrolyte imbalance is particularly common in hemodialysis patients, so it is required to monitor electrolyte changes between hemodialysis procedures in order to restore normal balance before arrhythmias occur.

The system includes a wearable device with integrated sensors for bio-signals (photoplethysmogram, electrocardiogram, bio-impedance, motions); modules for recognizing and monitoring electrolyte (potassium, calcium, magnesium, etc.) imbalance from the short electrocardiogram signal, while such modules are integrated into the wearable device; modules for recognizing and monitoring life-threatening cardiac arrhythmias (bradycardia, ventricular tachycardia, ventricular flutter, ventricular fibrillation), which combine signals of continuous photoplethysmogram and short electrocardiogram for integration into the wearable device; sudden death risk assessment module to be used on a server, PC, or smart device (e.g., smartphone, tablet, smart watch, etc.). The system and the method that are provided allow to execute a long-term monitoring of the life-threatening conditions in a patient-friendly way so it can be used both in hemodialysis facilities and at patient's home, especially during the long (3 days) interval between hemodialysis procedures.

In clinical practice electrolyte levels in blood serum are evaluated using invasive blood tests, however, in recent years focus has been on development of technologies capable to recognize electrolyte imbalances by non-invasive methods such as analyzing electrocardiogram signal on a body surface. Researchers at Mayo Clinic are developing an algorithm for measuring levels of potassium in blood serum based on analysis of surface electrocardiogram (ECG) by recording signals from glued-on-the-chest electrodes (Attia Z. et al. Novel bloodless potassium determination using a signal-processed single-lead ECG, Journal of the American Heart Association, 5(1), e002746, 2016), or by electrodes integrated into a smartphone case (Yasin O. Z. Et al. Noninvasive blood potassium measurement using signal-processed, single-lead ECG acquired from a handheld smartphone, Journal of Electrocardiology, 50, 620-625, 2017). The document US 8,948,854 B2 (published on 03/02/2015) describes a method and a system developed by Mayo Clinic investigators for non-invasive detection of changes in blood composition. Electrolyte (e.g., potassium, calcium, magnesium, etc.) fluctuations and physiological abnormalities are evaluated by analyzing cardiac cycle-averaged ECG signals patterns that can help distinguish pathological blood composition from normal. The document does not elaborate on the techniques of recording electrocardiograms. Also, it is not possible to associate changes in blood composition with the arrhythmia episodes and their risk. Document US 9,848,778 B2 (published on 26/12/2017) describes a method, a device and a system for monitoring condition of patients with kidney disease. Changes in blood potassium levels are detected by recognizing deviations in muscle or nerve cell activity from an electromyogram and/or ECG signal; also by analyzing changes in the T and R waveforms of the ECG signal; also by using electromyographic sensor for recording tissue response to the generated electrical pulse sequences. The device described in the patent assesses patient's risk of hyperkalemia, hypokalemia, and arrhythmias and may be used externally or implanted. The method uses only an ECG signal recorded from the patient's chest or by using invasive means and measures level changes of only one electrolyte (potassium) in the blood. Document US 9,289,165 B2 (published on 05/22/2016) describes a method and a device for monitoring changes in concentration levels of various ions (e.g., potassium, sodium, chlorine, calcium, magnesium) in a patient's extracellular fluid. Ion concentrations are monitored by electrically stimulating the patient's tissues (e.g., heart muscle, skeletal muscle, smooth muscle, nerve tissue, skin) and recording their response to the stimulation. The method described in the patent does not analyze the ECG signal waveform to detect electrolyte level changes in the blood serum.

Document US 9,554,725 B2 (published on 31/01/2017) describes a method and a device for measuring bioelectrical impedance, designed to monitor condition of patients having renal dysfunction in a non-invasive way during hemodialysis procedure. By analyzing bioelectrical impedance signals the system can determine how much fluid needs to be removed during hemodialysis procedure, the rate at which fluid is removed, as well as patient's hydration level and electrolyte balance. This described system does not provide monitoring of electrolyte fluctuations in daily living conditions.

To determine life-threatening arrhythmias, standard non-invasive Holter monitors and cardiac event recorders that use glued electrodes, or invasive devices such as implantable cardioverter-defibrillators are prescribed. Recent advances in electronics and medical technology has made it possible to detect arrhythmias using patient-friendly, minimal-contact techniques such as photoplethysmography, and record signals by a smartphone camera, a web camera, an in-ear sensor, or a smart wristband. Devices based on photoplethysmography for detection of arrhythmias have so far been limited to recognizing atrial fibrillation arrhythmias (US 6,519,490 B1, US 7,846,106 B2, US 9,433,386 B2) or ventricular extra systoles (US 7,794,406 B2, Solosenko A. et al. Photoplethysmography-based method for automatic detection of premature ventricular contractions, 9(5), 662-669, 2015). These cardiac arrhythmias per se are not categorized as life-threatening. The reliability of arrhythmia recognition in a photoplethysmogram signal recorded by sensors integrated in the wristband in normal daily living conditions is highly dependent on the signal quality which is often insufficient due to motion artifacts. Therefore, for improved reliability a method that involves combining both photoplethysmography and electrocardiogram may be applied (US 9,420,956 B2, US 9,839,363 B2, US 2017/0202459 A1, US 2015/0366518 A1). Wristband based on this method continuously record heart rate by integrated photoplethysmographic sensors and upon detection of atrial fibrillation trigger an alarm to inform the system user via an external device (US 9,420,956 B2), while biopotential sensors integrated in the wristband record a short electrocardiogram (US 9,839,363 B2). Function of an external electrocardiogram recorder can be accomplished by variety of devices such as smartphone case with integrated electrodes for recording bioelectric potentials, ECG recorder in the outpatient unit or clinic, etc.

Document US 2015/0366518 A1 (published on 24/12/2015) provides methods and a system for detecting and alerting individuals about significant and health-threatening situations (e.g., imminent myocardial infarction, stroke, epileptic seizure, etc.) and, if needed, activating the "panic" mode while remotely calling an ambulance. The system registers signals of photoplethysmogram, accelerometer and, if required, records short ECG. Various modifications of the device are specified including both wristbands and/or glasses with integrated photoplethysmogram sensors. The patent application does not describe methods for recognizing life-threatening arrhythmias. Document US 2017/0172424 A1 (published on 22/06/2017) describes a device for detecting life-threatening cardiac dysfunction leading to cardiac arrest. The device registers a photoplethysmogram signal that recognizes a life-threatening decrease in the heart rate and can automatically contact an ambulance; however, it is not designed to detect occurrences of life-threatening arrhythmias of any other type.

The prior solutions of analyzed technical level have following disadvantages compared to the solution presented in this description:
▪ The analyzed wearable systems cannot assess electrolyte fluctuations non-invasively; associate them with food intake and time of the day; and recognize pathological changes in electrolyte balance.
▪ The analyzed systems do not evaluate the association of electrolyte fluctuations with occurrences of life-threatening arrhythmia in general context.
▪ The analyzed wearable systems have no real-time detection of life-threatening cardiac arrhythmias (ventricular bradycardia, ventricular tachycardia, ventricular flutter, ventricular fibrillation).
▪ The systems analyzed do not assess risk of sudden death.
▪ The analyzed wearable systems have no ability to send real-time alarm to a physician in event of detection of life-threatening cardiac arrhythmias.

Technical solution presented in this description has no drawbacks listed above.

### BRIEF DESCRIPTION OF THE INVENTION

This description provides a system and method for recognizing and monitoring life-threatening health conditions, including electrolyte imbalance and life-threatening arrhythmias. Unlike the existing solutions the present invention is non-invasive and patient-friendly so life-threatening arrhythmias and electrolyte fluctuations can be monitored continuously - clinically and at home. Non-invasive technology for monitoring life-threatening medical conditions is useful in clinical monitoring of a patient (e.g., alerting hemodialysis staff about risk of arrhythmia) and at home as it allows to start treatment (e.g., to start hemodialysis earlier) before the occurrence of dangerous cardiac arrhythmias. The system is designed for hemodialysis facilities providing life-threatening monitoring services to their patients; also for patients after serious illnesses (myocardial infarction, cancer chemotherapy); also for patients on peritoneal dialysis who undergo dialysis on their own at home without continuous medical attention.

The system consists of a patient-worn device with integrated modules for real-time detection and monitoring of life-threatening conditions; a patient-operated smart device (e.g., smartphone, tablet, etc.) to monitor electrolyte fluctuations and enter additional information; a smart device (e.g., smartphone, tablet, smart watch, etc.) or personal computer used by a physician to analyze the summarized results; also server software modules for assessing risk of sudden death based on biosignal synergy. Life-threatening states are identified by photoplethysmogram, electrocardiogram, bioimpedance spectroscopy and motion signals. The wrist-worn device continuously records the photoplethysmogram that is used to identify life-threatening arrhythmias. A short (∼1 min.) electrocardiogram signal recorded by the wrist-worn device is used to indirectly monitor electrolyte fluctuations. To increase the number of electrocardiogram derivations (e.g., I, II, III, aVR, aVL, aVF) and to increase the reliability of the solutions the algorithms can be also used in other devices able to record electrocardiogram, such as smart scales with electrodes integrated in the handle. Data transfer from the patient's home is performed by using cloud technology through the patient's smart device. During long-term monitoring the physician is informed by email when life-threatening conditions are automatically detected.

### BRIEF DESCRIPTION OF THE DRAWINGS

Embodiments of the system are described in more detail below with reference to the accompanying figures that provide information as follows:
Figure 1 A diagram showing main components of the system and their interaction.
Figure 2 An illustration of the main components comprising a patient-worn device.
Figure 3 Characteristic examples of electrocardiogram and photoplethysmogram signals during life-threatening arrhythmias and possible values of temporary signal parameters allowing to identify different types of life-threatening arrhythmias.
Figure 4 One of possible methods of processing an electrocardiogram signal, allowing to obtain parameters for evaluating electrolyte fluctuations for application into a patient-worn device.
Figure 5 A concept of a graphical user interface of the patient's smart device (e.g., smartphone, tablet, etc.).
Figure 6 A concept of a graphical user interface of the physician's smart device (e.g., smartphone, tablet, etc.) or PC.

The presented figures are for illustration and the scale, the proportions and the other aspects do not necessarily correspond to the actual technical solution.

### DETAILED DESCRIPTION OF THE INVENTION

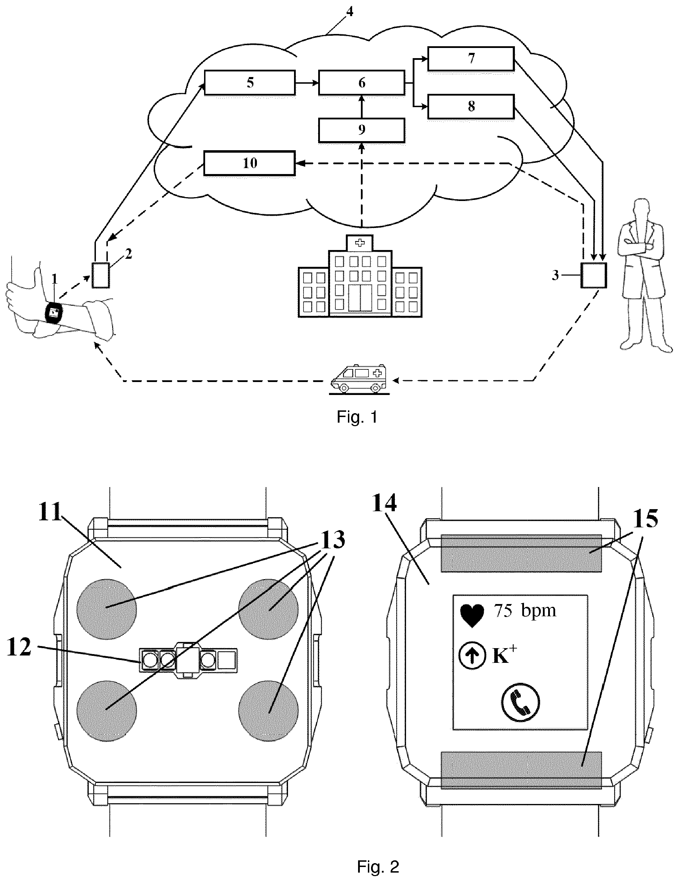

This description provides a system (Figure 1) and a method for patients with chronic kidney disease who have increased risk of life-threatening conditions (electrolyte fluctuations and cardiac arrhythmias as a result of thereof) but application is not limited to it. The concept of developing personalized decision support systems and techniques may also be adapted to the groups of other clinical pathologies that include episodes of electrolyte fluctuations and life-threatening arrhythmias such as heart failure, myocardial infarction, or after chemotherapy.

The system for recognizing and monitoring life-threatening conditions in a patient-friendly way comprises at least the following:
Equipment used by the patient:
   1. Patient-worn device (1);
   2. Patient's smart device (2);
Equipment used by the physician:
   3. Physician's smart device or personal computer (3).

In addition to the above mentioned devices the system also includes a server with an integrated sudden-death-risk-assessment module (4) that combines data aggregation and analysis units (5), expert system unit (6), alarm (7) and risk estimate presentation (8) units, patient data (9) and physician's recommendations (10). The server is equipped with technical measures to ensure the security of user data. The system also comprises an Internet network infrastructure, technical measures for interconnection of the listed devices and the ability to exchange data. Patient-worn device (1) is such a device that is capable of recognizing a life-threatening cardiac arrhythmias from a continuously recorded photoplethysmogram signal in real time which is also capable of performing other functions characteristic to such devices, e.g., instantaneous pulse recording, physical activity evaluation with integrated motion sensor, etc. The patient-worn device is usually fitted on a wrist but can also be mounted on other parts of the body (e.g., forearm, leg, head, ear, etc.). Function of such device can be performed by a smart wristband, a smart watch, a smart earpiece, a smart headband and other smart devices that can be used in this manner and have the said functions. The lower part (11) (Fig. 2) of the patient-worn device (1) has an integrated photoplethysmography sensor (12) for recording a continuous photoplethysmogram signal. Internal electrodes (13) are also integrated in the lower part (11) of the patient-worn device (1) (Fig. 2), while the outer electrodes (15) in the upper part (14) of the patient-worn device (1) are insulated from the inner electrodes (13). The inner electrodes (13) are in permanent contact with the skin of the wrist on which the device is placed, so that when the outer electrodes (15) are touched with the finger of the other hand, an electrocardiogram signal is recorded. An external device capable of exchanging signals and/or data with the patient's smart device (2) or server (4), such as a smart scale with integrated electrodes in their handles, may also be used to record a short electrocardiogram signal. The patient-worn device (1) may also include integrated sensors for recording bioimpedance and impedancegram signals, which are used to assess the patient's health. To ensure the quality of the recorded signals, capacitive sensors can be integrated into the patient-worn device (1), which utilizes the recorded signals in adaptive algorithms for removing artifacts from the photoplethysmogram signal and detecting poor sensor contact with the patient's skin. The patient-worn device (1) transmits signals and data to the server (4) during charging of the device via the USB port of a computer or similar device and/or wirelessly via the patient's smart device (2).

The patient's smart device (2) is designed to ensure data exchange between the patient-worn device and the system server. The patient's smart device (2) enables the patient to monitor his or her state of health and enter relevant information for the physician, as well as to receive physician's instructions and reminders important for maintaining a stable state of the patient's health. The patient's smart device (2) can also perform processing of signals recorded by the patient-worn device (1) and recognition of health-threatening conditions. The functions of the patient's smart device (2) can be performed by various smart devices (e.g., smartphone, tablet, smart watch, etc.) equipped with technical means of communication with the internet, having installed system software enabling data exchange with the system server (4).

The physician's smart device (3) is designed to provide access for the physician to the patient's health data on the system server (4). The physician's smart device (3) enables the physician to obtain the detailed information about the patient's behavior and his/her medical condition, on the basis of which the physician makes a decision by using his/her smart device. The functions of the physician's smart device (3) can be performed by various smart devices (e.g., smartphone, tablet, smart watch, etc.) equipped with technical means of communication with the internet and having installed system software enabling data exchange with the system server (4).

### Description of the method

The patient's life-threatening conditions due to electrolyte fluctuations and arrhythmias are evaluated in the system as described below. Different signals are used to monitor electrolyte fluctuations and arrhythmias, but the patient's health status is evaluated by combining the results of analysis of these signals. Relationships between electrolyte fluctuations and arrhythmias are identified and used to increase the sensitivity and specificity of the system when detecting patient's life-threatening conditions.

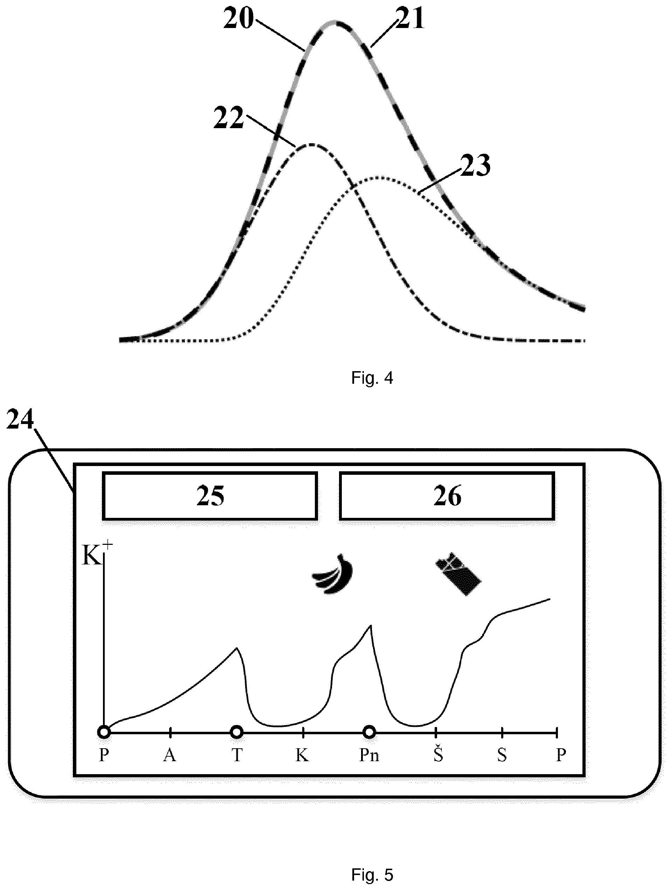

In the patient-worn device (1) or the separate smart device (2) that acts as its controller, software modules are installed for detection of life-threatening cardiac arrhythmias from the photoplethysmogram signal (Figure 4), which:
▪ Analyzes the real-time photoplethysmogram signal and detects the life-threatening cardiac arrhythmias.
▪ Automatically recognizes the life-threatening arrhythmias and informs the system user to touch the electrodes integrated into the worn device and thereby record a short electrocardiogram signal for confirming the arrhythmia.
▪ After recognizing the life-threatening cardiac arrhythmias, sends data to the server for further analysis to create the reports.
▪ In the event of a prolonged life-threatening arrhythmia, sends an alarm to the doctor's smart device.

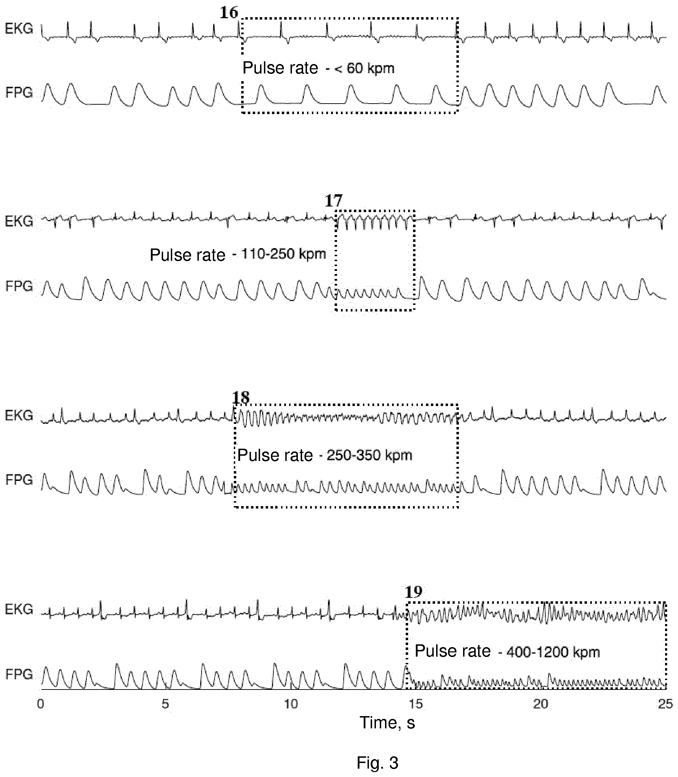

The algorithm for recognition of life-threatening cardiac arrhythmias and long-term monitoring of the progression of arrhythmias is based on the analysis of temporal and amplitude changes in photoplethysmogram signal parameters (Fig. 3). Life-threatening arrhythmias are recognized by distinctive features of their individual type, e.g. bradycardia (16) is recognized by a significantly reduced heart rate, dropping to 60 beats or less per minute; ventricular tachycardia (17) - by a regular rhythm of 110 to 250 beats per minute with the sudden start and sudden end and the steady-height, reduced, pulse amplitude; ventricular flutter (18) - by irregular rhythm of 250 to 350 beats per minute and variable-height, reduced, pulse amplitude, ventricular fibrillation (19) - by irregular rhythm, reaching more than 400 beats per minute and very low pulse amplitude. The number of false alarms due to motion artifacts and other, less significant, rhythm disturbances can be reduced by taking into account information provided by other recorded signals, such as electrolyte fluctuations. If electrolyte imbalance is detected, the sensitivity threshold for arrhythmia recognition may be lowered, and in case of normal electrolyte balance it can be raised. For detecting the life-threatening arrhythmias, the patient-worn device (1) can additionally record and analyze an impedance signal.

Patient-worn device (1) or the separate smart device (2) that acts as its controller includes software modules to assess electrolyte fluctuations from the electrocardiogram signals (Fig. 3), which:
▪ Automatically inform the system user at specified intervals to record a short electrocardiogram signal to assess electrolyte fluctuations.
▪ After assessing electrolyte fluctuations, sends the data to the server to collect, visualize, analyze, create reports and inform physicians.
▪ In the event of a health-threatening electrolyte imbalance, sends an alarm to the doctor's smart device.

Electrolyte (potassium, calcium, magnesium, etc.) fluctuations are assessed by distinguishing the signs of waveforms, amplitudes and durations from the short electrocardiogram signal (Fig. 4). These signs may be assessed by using a single-cardiac-cycle electrocardiogram or by averaging multiple cardiac cycle electrocardiograms. Symptoms can be distinguished directly from the electrocardiogram signal (20) (amplitude, duration, slope, etc. of individual waveforms). By increasing noise resistance, signs can also be distinguished indirectly from models (21) matched to electrocardiogram signal waveforms (normal (22), lognormal (23), or other function parameters). Electrolyte fluctuations can be detected from a short electrocardiogram signal recorded not only by the patient's smart device (1), but also by other device, such as smart scales. The reliability of electrolyte fluctuation detection can be enhanced by calibrating the method according to pre- and post-dialysis blood tests.

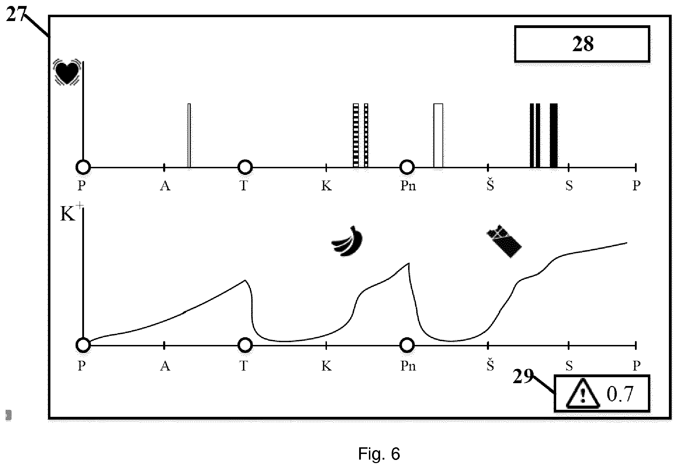

The server software module (4) analyzes the long-term distribution and progress profile of the life-threatening states recorded by the worn device (1) and provides parameters of electrolyte fluctuations and arrhythmia episodes characterizing their relationship, predicts the life-threatening states of the system user, the patient, and assesses the risk of sudden death. The server software module (4) also provides the ability to exchange data between devices, visualize data and generate reports. Via the internet connection (https and/or other technologies), the patient's smart device (2) and the doctor's smart device or personal computer (3) are connected to the server. The patient's smart device receives information about the physician's decision that the physician makes after the physician's smart device (3) receives information processed on the server (4). Graphical user interface (Figure 5) (24) of the patient's smart device enables the patient to monitor electrolyte fluctuations, self-enter physician-relevant information (25), and receive reminders important for maintaining a stable patient status (26). Through the graphical user interface of the physician's smart device (Figure 6) (27) the physician obtains detailed information on the occurrence and progress of life-threatening arrhythmias during long-term monitoring and their relationship to electrolyte fluctuations, patient behavior (e.g., physical activity, body position, etc.)., food intake, physiological state (e.g., sleep, stress), etc. Through the graphical user interface of the smart device the physician can perform a detailed analysis of the patient's condition (28) and see an estimation of the patient's risk of sudden death (29).

In order to illustrate and describe the present invention, description of preferred embodiments is provided here. This description is not detailed or restrictive, which means it does not seek to provide the exact form or embodiment. The above description should be viewed as an illustration rather than a limitation. It is clear that many modifications and variations may be apparent to those skilled in this field. This embodiment is selected and described to enable those skilled in the field to understand the principles of the present invention and their best practical application for different embodiments with different modifications suitable for a particular application or embodiment. The scope of this invention shall be defined by the appended Claims and their equivalents, where all mentioned terms are intended to have a broad meaning unless indicated otherwise.

In the embodiments described by those skilled in the field, modifications may be made within the scope of the present invention as defined in the following Claims.

## Claims

1. A system for long-term monitoring and recognizing life-threatening conditions in a patient-friendly way comprising a patient-worn device (1), a patient's smart device (2), a doctor's smart device or a personal computer (3) and a server (4) for transmitting data between devices, data storing, data processing, visualizing and generating reports **characterized in that** the patient wearable device (1) registers a continuous photoplethysmogram signal with an integrated photoplethysmogram sensor (12) and a transient electrocardiogram signal is registered with integrated biopotential sensors (13, 15) in the patient wearable device (1).

2. A system for monitoring and recognizing life-threatening conditions in a patient-friendly way according to the preceding claim **characterized in that** the patient-worn device (1) registers movements with an integrated sensor.

3. A system for monitoring and recognizing life-threatening conditions in a patient-friendly way according to the preceding claims **characterized in that** the patient-worn device (1) registers bioimpedance with an integrated sensor (13, 15).

4. A system for monitoring and recognizing life-threatening conditions in a patient-friendly way according to the preceding claims **characterized in that** the patient-worn device (1) registers an impedance signal with an integrated sensor (13).

5. A system for monitoring and recognizing life-threatening conditions in a patient-friendly way according to the preceding claims **characterized in that** capacitive sensors are integrated in the patient-worn device (1).

6. A system for monitoring and recognizing life-threatening conditions in a patient-friendly way according to the preceding claims **characterized in that** the short electrocardiogram is recorded using bio-potential electrodes integrated into smart scales.

7. A method implemented in the system according to Claims 1-6 for long-term monitoring and recognizing life-threatening conditions in a patient-friendly way **characterized in that** the patient's smart device (1) registers a continuous photoplethysmogram signal for real-time recognition of life-threatening arrhythmias (16, 17, 18, 19); the patient at defined intervals is informed to record a short electrocardiogram signal which is used to monitor electrolyte fluctuations (20, 21, 22, 23); data about life-threatening arrhythmias and electrolyte fluctuations are sent to a server (4) and analyzed in it; the patient and the physician are informed through their smart devices (2, 3) about the patient's health status, based on the occurrence of life-threatening arrhythmias and electrolyte fluctuations, the risk of sudden death is assessed; in case of occurrence of life-threatening condition due to a prolonged life-threatening arrhythmia episode and/or if electrolyte imbalance that is hazardous to health is detected an alert is urgently sent to the physician or other persons closely associated with the patient.

8. A method for monitoring and recognizing life-threatening conditions in a patient-friendly way according to Claim 7 **characterized in that** detection of life-threatening arrhythmias is further supported by an impedance signal analysis.

9. A method for monitoring and recognizing life-threatening conditions in a patient-friendly way according to Claims 7-8 **characterized in that** upon detection of a life-threatening arrhythmia episode in the continuously recorded signal by the patient's smart device (1) the patient is informed to record a short electrocardiogram signal which after analysis is used to confirm the arrhythmia episode.

10. A method for monitoring and recognizing life-threatening conditions in a patient-friendly way according to Claims 7-9 **characterized in that** for assessing electrolyte fluctuations a short electrocardiogram is recorded by using biopotential electrodes embedded in a smart wristband or a smart scale.

11. A method for monitoring and recognizing life-threatening conditions in a patient-friendly way according to Claims 7-10 **characterized in that** the system user is provided with an indication of an increase in electrolyte imbalance and its association with food intake and used medications (24) that may influence electrolyte imbalance to the patients with chronic kidney disease.

12. A method for monitoring and recognizing life-threatening conditions in a patient-friendly way according to Claims 7-11 **characterized in that** a bioimpedance sensor (13, 15) is used to detect an increase in fluid content in the body.

13. A method for monitoring and recognizing life-threatening conditions in a patient-friendly way according to Claims 7-12 **characterized in that** the physician is informed about the patient's electrolyte imbalance the distribution and type of life-threatening arrhythmia episodes (27) and the risk of patient's life-threatening conditions (29).

14. A method for monitoring and recognizing life-threatening conditions in a patient-friendly way according to Claims 7-13 **characterized in that** the artifacts in the photoplethysmogram signal are removed by adaptive algorithms while using capacitive sensors to register signals.
